# EUROPÄISCHE PATENTSCHRIFT

(11) **EP 1 227 749 B1**
(45) Veröffentlichungstag und Bekanntmachung des Hinweises auf die Patenterteilung: **09.07.2003**
(21) Anmeldenummer: 00977503.2
(22) Anmeldetag: 08.11.2000
(51) Int. Cl.: A61B 1/32

(54) **SPREIZBARES MEDIZINISCHES INSTRUMENT, INSBESONDERE LARYNGOSKOP**
MEDICAL INSTRUMENT THAT CAN BE SPREAD, ESPECIALLY A LARYNGOSCOPE
INSTRUMENT MEDICAL A PARTIE ECARTABLE, EN PARTICULIER UN LARYNGOSCOPE

(30) Priorität: 11.11.1999 DE 19954442
(43) Veröffentlichungstag der Anmeldung: 07.08.2002
(73) Patentinhaber: Karl Storz GmbH & Co. KG, 78532 Tuttlingen (DE)
(72) Erfinder: STIHL, Ewald, 78187 Geisingen (DE)
(74) Vertreter: Hofmeister, Frank Horst
(86) Internationale Anmeldenummer: EP0010995
(87) Internationale Veröffentlichungsnummer: WO01034019

(56) Entgegenhaltungen:
- US-A- 3 841 317

## Beschreibung

Die Erfindung betrifft ein spreizbares medizinisches Instrument mit einem Griffteil und mit mindestens zwei mit dem Griffteil verbundenen Spatelteilen, die über wenigstens einen Einstellmechanismus zwischen einer Ausgangsstellung, in der die Spatelteile aneinanderliegen und wenigstens einer Arbeitsstellung parallel und/oder winklig gegeneinander verstellbar sind, wobei die einzelnen Spatelteile in der Arbeitsstellung zumindest im distalen Bereich seitliche Spalte bildend voneinander beabstandet sind und diese seitlichen Spalte zwischen den Spatelteilen in der Arbeitsstellung der Spatelteile über verschwenkbare Klappenelemente zumindest weitestgehend abdeckbar sind.

Laryngoskope werden insbesondere zur direkten instrumentellen Inspektion des Kehlkopfes sowie endolaryngeale chirurgische Eingriffe verwendet. Grundsätzlich lassen sich die Laryngoskope in starre und spreizbare Laryngoskope unterteilen. Bei einem starren Laryngoskop ist der mit dem Griffteil verbundene Spatelteil als geschlossenes oder eventuell einseitig geschlitztes hohles, im wesentlichen kreiszylindrisches Rohr ausgebildet, das ein konstantes Lumen zum Einführen geeigneter medizinischer Instrumente aufweist. Nachteilig bei den starren Laryngoskopen ist, daß sich einerseits das Lumen bei Bedarf nicht vergrößern läßt und es andererseits notwendig ist, das Laryngoskop mit seinem unveränderbaren Lumen in den Mund- und Rachenraum des intubierten und narkotisierten Patienten einzuführen.

Diese Probleme-können durch die Verwendung spreizbarer Laryngoskope vermieden werden. Diese Laryngoskope weisen ein aus wenigstens zwei gegeneinander verstellbaren Spatelteilen bestehendes, mit dem Griffteil verbundenes Spatelteil auf, dessen Lumen zwischen einer Ausgangsstellung zum Einführen und Entfernen des Laryngoskops, in der die einzelnen Spatelteile so eng wie möglich aneinanderliegen, und einer Arbeitsstellung verstellbar ist, in der die einzelnen Spatelteile parallel und/oder winklig gegeneinander verstellt sind. Beim parallelen und/oder winkligen verstellen der einzelnen Spatelteile gegeneinander entstehen zumindest im distalen Bereich der Spatelteile Spalte zwischen den einzelnen Spatelteilen. Die Ausbildung dieser Spalte ist aber nachteilig, da die Möglichkeit besteht, daß durch diese seitlichen Spalte Weichteile, wie beispielsweise Wange oder Zunge in den Innenraum des Laryngoskops gelangen, wodurch die Gefahr einer Verletzung dieser Weichteile besteht und auch der Arbeitsraum für den Operateur deutlich verringert wird.

Um das Eindringen von Weichteilen in einspreizbares medizinisches Instrument während der Operation zu vermeiden, ist es in der Praxis üblich, mit einem zusätzlichen Spatel von der Innenseite des Instruments her diese seitlichen Spalte zuzuhalten. Zusätzlich hat es sich als hilfreich erwiesen, die äußere Oberfläche der Spatelteile aufgerauht auszugestalten, wodurch beispielsweise ein seitliches Abgleiten der Zunge vermieden werden kann. Die Verwendung eines zusätzlichen Spatels verringert das dem Operateur zur Verfügung stehende freie Lumen deutlich und verlangt zusätzlich die Hilfe eines Assistenten. Die aufgerauhte äußere Oberfläche wiederum erschwert das Reinigen des medizinischen Instruments, weshalb auch diese Ausgestaltung als in gewissen Maße nachteilig anzusehen ist.

Ein gattungsgemäßes spreizbares medizinisches Instrument ist aus der US 5,868,668 A bekannt. Bei diesem bekannten medizinischen Instrument sind die Klappenelemente zum Abdecken der seitlichen Spalte so verschwenkbar am Griffteil gelagert, daß diese in der Ausgangsstellung der Spatelteile zwischen den Spatelteilen angeordnet sind. Erst, wenn die Spatelteile in eine erste Spreizstellung geöffnet worden sind, können bei einem weiteren Öffnen der Spatelteile die Klappelemente in ihre die seitlichen Spalte zumindest teilweise abdeckende Stellung verschwenkt werden. Da aber während des ersten Spreizens der Spatelteile die Klappenelemente nicht betätigbar sind, kann Gewebe zwischen die Spatelteile gelangen und den Arbeitsraum für den Operateur verringern. Weiterhin ist es bei diesem bekannten Instrument nachteilig, daß der Operateur das Verschwenken der Klappenteile manuell durch das weitere Spreizen der Spatelteile einleiten muß und darüber hinaus auch das Offenhalten der Spatelteile einer fortwährenden manuellen Betätigung bedarf.

Aus der WO 98/33431 ist es weiterhin bekannt, über die gegeneinander zu verstellenden Spatelteile eine Hülse aus einem elastischen Material zu stülpen, das sich beim Verstellen der einzelnen Spatelteile gegeneinander dehnt und so die seitlichen Spalte zwischen den Spatelteilen verschließt. Diese Art die seitlichen Spalte zu verschließen hat den Nachteil, daß durch das dehnbare elastische Material, das die Spatelteile umschließt, der Kraftaufwand zum gegenseitigen verstellen der Spatelteile zusätzlich zum Druck des umgebenden Gewebes deutlich erhöht wird und die Spatelteile permanent in die Schließrichtung (Ausgangsstellung) druckbelastet sind. Weiterhin besteht die Gefahr, daß beim Einführen der Operationsinstrumente sowie beim Operieren mittels Laser das elastische Material beschädigt wird und somit die Schutzwirkung nicht mehr oder nicht mehr vollständig vorliegt.

Ausgehend von diesem Stand der Technik liegt der Erfindung die **Aufgabe** zugrunde, ein spreizbares medizinisches Instrument der eingangs genannten Art so weiterzubilden, daß dieses bei einfacher Handhabung auf einfache Art und Weise zuverlässig das Eindringen von Weichteilen durch die seitlichen Spalte zwischen die gespreizten Spatelteile verhindert.

Die **Lösung** dieser Aufgabenstellung ist erfindungsgemäß dadurch gekennzeichnet, daß die verschwenkbaren Klappenelemente jeder Seite jeweils an zumindest einem der Spatelteil derart festgelegt sind, daß sie in der Ausgangsstellung der Spatelteile formschtüssig an der Außenseite des jeweils anderen, nicht mit dem jeweiligen Klappenelement versehenen Spatelteil anliegen.

Durch die erfindungsgemäße Festlegung der Klappenelemente einer jeden Seite an jeweils zumindest einem Spatelteil wird sichergestellt, daß die Klappenelemente von Anbeginn der Spreizbewegung der Spatelteile bereitstehen und die sich bildenen seitlichen Spalte sofort abdecken, ohne, daß es hierzu einer separaten Betätigung der Klappenelemente bedarf. Da die Klappenelemente in der Ausgangsstellung formschlüssig an den Spatelteilen anliegen und mit den sich spreizenden Spatelteilen in die die Spalte abdeckende Stellung verschwenkt werden, bietet ein solchermaßen ausgestaltetes Instrument dem Operateur somit jederzeit einen freien Arbeitsquerschnitt.

Aufgrund des formschlüssigen Anliegens der Klappenelemente an den Spatelteilen wird das Einführen und Entfernen des spreizbaren medizinischen Instruments in der Ausgangsstellung erleichtert, da dadurch der Außendurchmesser des medizinischen Instruments gegenüber einem Instrument ohne Klappenelemente nur unwesentlich vergrößert wird, aber immer noch deutlich geringer ist als bei einem starren Endoskop mit vergleichbarem Arbeitsquerschnitt.

Gemäß einer praktischen Ausführungsform wird weiterhin vorgeschlagen, daß das Verschwenken der Klappenelemente automatisch über die gegeneinander verstellbaren Spatelteile erfolgt. Durch dieses automatische Verschließen der seitlichen Spalte bedarf es keiner zusätzlichen Handgriffe oder anderweitiger Maßnahmen, wodurch der Operateur entlastet wird.

Vorteilhafterweise wir weiterhin vorgeschlagen, daß die Klappenelemente lösbar an den Spatelteilen festgelegt sind, was die Reinigung zusätzlich vereinfacht.

Bei einer bevorzugten Ausführungsform eines spreizbaren medizinischen Instruments mit zwei gegeneinander verstellbaren Spatelteilen wird vorgeschlagen, daß ein Spatelteil starr mit dem Griffteil verbunden ist und das andere Spatelteil parallel und winklig in Bezug auf das starre Spatelteil verstellbar mit dem Griffteil verbunden ist, und daß jeweils ein verschwenkbares Klappenelement an einer Längsseite des starren Spatelteils angeordnet ist. Die Ausbildung eines starren und eines verstellbaren Spatelteils ermöglicht einen besonders einfach aufgebauten Einstellmechanismus zum Verstellen der Lage der Spatelteile zueinander.

Die Betätigung der Klappenelemente kann dadurch erleichtert werden, daß diese in die an den Spatelteilen anliegende Ausgangsstellung federbelastet sind. Dadurch wird sichergestellt, daß sich die Klappenelemente sofort wieder formschlüssig an die Spatelteile anlegen, wenn diese wieder aufeinander zu verstellt werden.

Die Höhe der erfindungsgemäßen verschwenkbaren Klappenelemente ist so bemessen, daß zumindest ein Teil eines jeden Klappenelements in der Arbeitsstellung der Spatelteile an der äußeren Oberfläche der nicht mit dem jeweiligen Klappenelement versehenen Spatelteil anliegt. Auf diese Weise wird sichergestellt, daß die Klappenelemente nicht nach innen zwischen die Spatelteile klappen können, wodurch ein Zurückverstellen der Spatelteile in die Ausgangsstellung blockiert werden könnte.

Weiterhin wird mit der Erfindung vorgeschlagen, daß die äußeren Oberflächen der Spatelteile und/oder der Klappenelemente glatt ausgebildet sind. Aufgrund der Verwendung der Klappenelemente, die die seitlichen Spalte verschließen, kann auf die Aufrauhung der äußeren Oberflächen verzichtet werden, weshalb ein solchermaßen ausgestaltetes spreizbares medizinisches Instrument einfach und gründlich zu reinigen ist.

Schließlich wird mit der Erfindung vorgeschlagen, daß das erfindungsgemäße spreizbare medizinische Instrument ein Laryngoskop ist.

Weitere Merkmale und Vorteile der Erfindung ergeben sich aus der nachfolgenden Beschreibung der zugehörigen Zeichnung, in der nur beispielhaft eine Ausführungsform eines als Laryngoskop ausgebildeten erfindungsgemäßen spreizbaren medizinischen Instruments dargestellt ist. In der Zeichnung zeigt:
- Fig. 1: eine Seitenansicht eines erfindungsgemäßen Laryngoskops in der Ausgangsstellung;
- Fig. 2: eine Ansicht des Laryngoskops gemäß Fig. 1 von hinten;
- Fig. 3: eine ausschnittweise Vorderansicht des Laryngoskops gemäß Fig. 1 und 2, jedoch eine Arbeitsstellung darstellend;
- Fig. 4: eine schematische Seitenansicht der Spatelteile in der Arbeitsstellung mit parallel zueinander verstellten Spatelteilen und
- Fig.5: eine schematische Seitenansicht gemäß Fig. 4, jedoch mit winklig zueinander verstellten Spatelteilen.

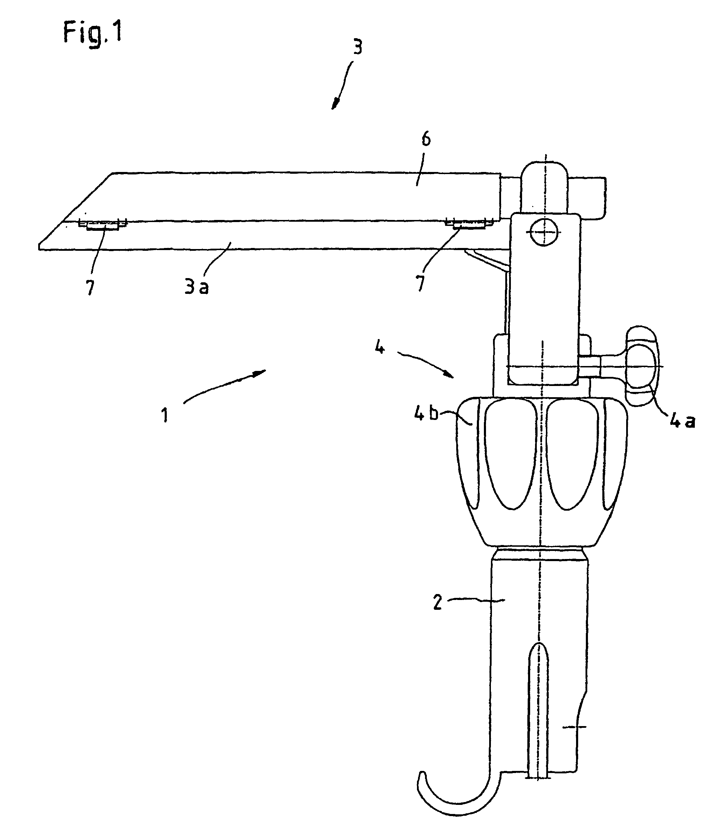

Die Abbildung Fig. 1 zeigt in der Seitenansicht ein Laryngoskop 1, welches im wesentlichen aus einem Griffteil 2 und einem Spatelteil 3 besteht. Bei dem dargestellten Laryngoskop 1 handelt es sich um ein spreizbares Laryngoskop 1, dessen Spatelteil 3 aus einem starren Spatelteil 3a und einem gegenüber dem starren Spatelteil 3a verstellbaren Spatelteil 3b besteht. Das Verstellen der Spatelteile 3a und 3b gegeneinander erfolgt bei diesem Laryngoskop 1 über einen aus zwei Stellschrauben 4a und 4b bestehenden Einstellmechanismus 4.

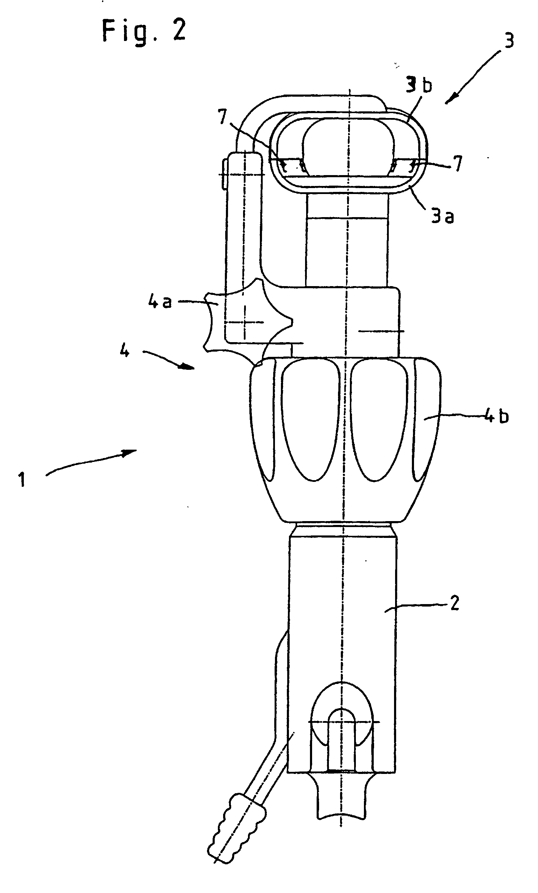

Die Abbildungen Fig. 1 und Fig. 2 zeigen das Laryngoskop in der Ausgangsstellung, in der die Spatelteile 3a und 3b so eng wie möglich aneinanderliegen, um den Spatelteil 3 des Laryngoskops 1 möglichst gut in den Mund- und Rachenraum eines Patienten einführen bzw. aus diesem wieder entfernen zu können. Das verbleibende Lumen des Spatelteils 3 in der Ausgangsstellung ist ausreichend, um mittels eines angesetzten Lichtträgers die Lage des Laryngoskops 1 im Rachenraum des Patienten zu bestimmen.

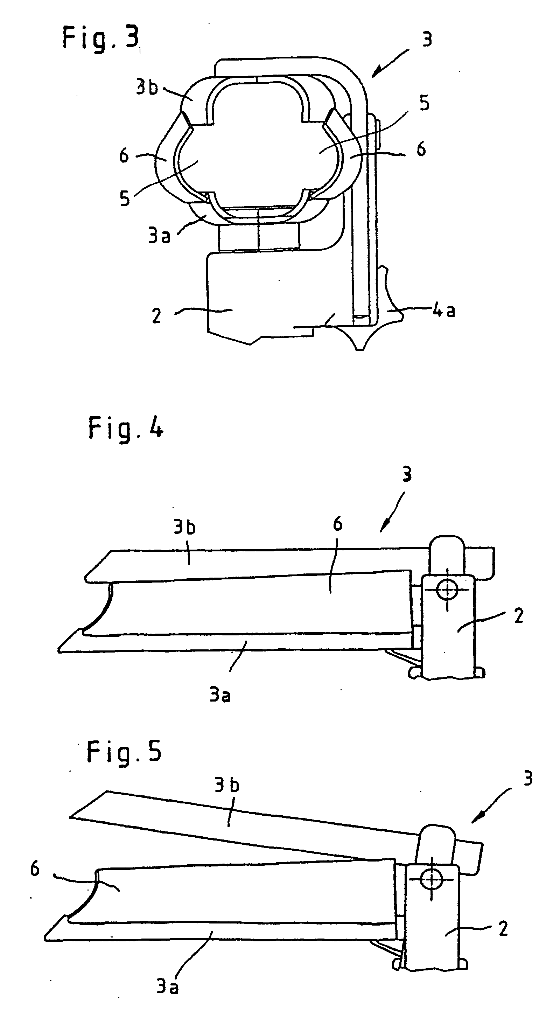

Um zu verhindern, daß durch seitliche Spalte 5, die sich zwischen den Spatelteilen 3a und 3b in der in den Abbildungen Fig. 3 bis Fig. 5 dargestellten Arbeitsstellung ergeben, Weichteile, wie beispielsweise Wange oder Zunge in das Innere des Laryngoskops 1 gelangen, wodurch die Gefahr einer Verletzung dieser Weichteile besteht und das freie Lumen für den Operateur deutlich verringert wird, weist das dargestellte Laryngoskop 1 am starren Spatelteil 3a verschwenkbar angeordnete Klappenelemente 6 auf, die die Spalte 5 in der Arbeitsstellung zumindest weitestgehend abdecken.

Die bei diesem Ausführungsbeispiel über Scharniere 7 verschwenkbar an dem starren Spatelteil 3a festgelegten Klappenelemente 6 liegen in der Ausgangsstellung der Spatelteile 3a, 3b im wesentlichen formschlüssig an der Außenseite des verstellbaren Spatelteils 3b an, so daß die Außenkontur des Spatelteils 3 durch die Klappenelemente 6 nur geringfügig vergrößert wird.

Nach dem Einführen des Spatelteils 3 des Laryngoskops 1 in den Rachenraum des Patienten werden die Spatelteile 3a und 3b entweder parallel, siehe Fig. 3 und Fig. 4, und/oder aber winklig, siehe Fig. 5, gegeneinander verstellt, um das Lumen des Spateiteils 3 zu vergrößern, um ausreichend Raum und Bewegungsfreiheit im distalen Bereich für die Operation zu schaffen. Durch das Auseinanderbewegen der Spatelteile 3a und 3b werden die Klappenelemente 6 automatisch aus der in Fig. 1und Fig. 2 dargestellten Ausgangsstellung so nach außen gedrückt, daß sie in der in Fig., 3 dargestellten Arbeitsstellung die seitlichen Spalte 5 zwischen den Spatelteilen 3a und 3b verschließen.

Neben der dargestellten Ausführungsform, bei der die Klappenelemente 6 nur an einem Spatelteil, nämlich dem starren Spatelteil 3a , befestigt sind, ist es selbstverständlich auch möglich, die Klappenelemente 6 am verschwenkbaren Spatelteil 3b oder auf einer Seite am Teil 3a und auf der anderen Seite am Teil 3b gelenkig zu lagern.

Fig. 5 zeigt das Spatelteil 3 in einer Arbeitsstellung, in der die Spatelteile zusätzlich winklig gegeneinander verschwenkt sind. Um einen einwandfreien Betrieb eines solchermaßen ausgebildeten Laryngoskops 1 gewährleisten zu können, muß sichergestellt sein, daß die Höhe der einzelnen Klappenelemente 6 so bemessen ist, daß zumindest ein Teil der Klappenelemente 6 in der Arbeitsstellung der Spatelteile 3a, 3b an der äußeren Oberfläche des nicht mit dem jeweiligen Klappenelement 6 verbundenen Spatelteil 3b anliegt. Andernfalls könnten die Klappenelemente 6 zwischen die Spatelteile 3a, 3b geraten, was zu Komplikationen beim Verstellen der Spatelteile 3a, 3b in die Ausgangsstellung führen würde.

Nach der Operation werden die Spatelteile 3a, 3b wieder zurück in die Ausgangsstellung verstellt, wobei sich die Klappenelemente 6 automatisch wieder formschlüssig an die Außenseite des Spatelteils 3b anlegen. Das Zurückstellen der Klappenelemente 6 kann dadurch unterstützt werden, daß die Klappenelemente 6 in die an dem Spatelteil 3b anliegende Ausgangsstellung federbelastet sind.

Ein solchermaßen ausgestaltetes spreizbares medizinisches Instrument zeichnet sich dadurch aus, daß durch die verschwenkbar an zumindest einem der Spatelteile 3a, 3b gelagerten Klappenelemente 6 in der Arbeitsstellung die Spalte 5 zwischen den Spatelteilen 3a, 3b so sicher verschlossen werden, daß keine Gefahr besteht, daß Weichteile, wie beispielsweise die Wange oder Zunge zwischen die Spatelteile 3a und 3b geraten können. Aufgrund dieser sicheren Abdichtung der Spalte 5 können die äußeren Oberflächen der Spatelteile 3a und 3b sowie der Klappenelemente 6 möglichst glatt ausgebildet werden, da eine aufgerauhte Oberfläche dieser Teile zum Zurückhalten der Zunge, wie dies aus der Praxis bekannt ist, nicht mehr notwendig ist. Ein solchermaßen ausgestaltetes Laryngoskop 1 läßt sich somit einfach und gründlich reinigen.

Neben der dargestellten Verwendung des spreizbaren medizinischen Instruments als Laryngoskop ist diese Ausgestaltung für verschiedene endoskopische Einsatzzwecke, wie beispielsweise bei der Wirbelsäulenchirurgie sowie der plastischen Chirurgie verwendbar, da auch hier Weichteile am Eindringen in den spreizbaren endoskopischen Zugang gehindert werden sollen. Vorteil des spreizbaren Zugangs ist der verhältnismäßig große distal zur Verfügung stehende Arbeitsbereich bei einem vergleichsweise geringen Außenumfang in der Ausgangsstellung beim Einführen bzw. Entfernen.

### Bezugszeichenliste

- 1: Laryngoskop
- 2: Griffteil
- 3: Spatelteil
- 3a: starres Spatelteil
- 3b: verstellbares Spatelteil
- 4: Einstellmechanismus
- 4a: Stellschraube
- 4b: Stellschraube
- 5: Spalt
- 6: Klappenelement
- 7: Scharnier

## Patentansprüche

1. Spreizbares medizinisches Instrument mit einem Griffteil (2) und mit mindestens zwei mit dem Griffteil (2) verbundenen Spatelteilen (3a, 3b), die über wenigstens einen Einstellmechanismus (4) zwischen einer Ausgangsstellung, in der die Spatelteile (3a, 3b) aneinanderliegen und wenigstens einer Arbeitsstellung parallel und/oder winklig gegeneinander verstellbar sind, wobei die einzelnen Spatelteile (3a, 3b) in der Arbeitsstellung zumindest im distalen Bereich seitliche Spalte (5) bildend voneinander beabstandet sind und diese seitlichen Spalte (5) zwischen den Spatelteilen (3a, 3b) in der Arbeitsstellung der Spatelteile (3a, 3b) über verschwenkbare Klappenelemente (6) zumindest weitestgehend abdeckbar sind,
**dadurch gekennzeichnet,**
**daß** die verschwenkbaren Klappenelemente (6) jeder Seite jeweils an zumindest einem der Spatelteil (3a, 3b) derart festgelegt sind, daß sie in der Ausgangsstellung der Spatelteile (3a, 3b) formschlüssig an der Außenseite des jeweils anderen, nicht mit dem jeweiligen Klappenelement (6) versehenen Spatelteil (3b, 3a) anliegen.

2. Spreizbares medizinisches Instrument nach Anspruch 1, **dadurch gekennzeichnet, daß** das Verschwenken der Klappenelemente (6) automatisch über die gegeneinander verstellbaren Spatelteile (3a, 3b) erfolgt.

3. Spreizbares medizinisches Instrument nach Anspruch 1 oder 2, **dadurch gekennzeichnet, daß** die Klappenelemente (6) lösbar an den Spatelteilen festgelegt sind.

4. Spreizbares medizinisches Instrument nach mindestens einem der Ansprüche 1 bis 3 mit zwei gegeneinander verstellbaren Spatelteilen (3a, 3b), **dadurch gekennzeichnet, daß** ein Spatelteil (3a) starr mit dem Griffteil (2) verbunden ist und das andere Spatelteil (3b) in Bezug auf das starre Spatelteil (3a) parallel und winklig verstellbar mit dem Griffteil (2) verbunden ist, und daß jeweils ein verschwenkbares Klappenelement (6) an einer Längsseite des starren Spatelteils (3a) angeordnet ist.

5. Spreizbares medizinisches Instrument nach mindestens einem der Ansprüche 1 bis 4, **dadurch gekennzeichnet, daß** die verschwenkbaren Klappenelemente (6) in die an den Spatelteilen (3a, 3b) anliegende Ausgangsstellung federbelastet sind.

6. Spreizbares medizinisches Instrument nach mindestens einem der Ansprüche 1 bis 5, **dadurch gekennzeichnet, daß** die Höhe der verschwenkbaren Klappenelemente (6) so bemessen ist, daß zumindest ein Teil eines jeden Klappenelements (6) in der Arbeitsstellung der Spatelteile (3a, 3b) an der äußeren Oberfläche des nicht mit dem jeweiligen Klappenelement (6) versehenen Spatelteil (3b) anliegt.

7. Spreizbares medizinisches Instrument nach mindestens einem der Ansprüche 1 bis 6, **dadurch gekennzeichnet, daß** die äußeren Oberflächen der Spatelteile (3a, 3b) und/oder der Klappenelemente (6) glatt ausgebildet sind.

8. Spreizbares medizinisches Instrument nach mindestens einem der Ansprüche 1 bis 7, **dadurch gekennzeichnet, daß** das Instrument ein Laryngoskop (1) ist.

## Claims

1. Spreadable medical instrument with a grip part (2) and with at least two spatula parts (3a, 3b) which are connected to the grip part (2) and which, via at least one adjusting mechanism (4), can be adjusted in parallel and/or at an angle to one another between an initial position, in which the spatula parts (3a, 3b) bear on one another, and at least one working position, the individual spatula parts (3a, 3b) being spaced apart from one another in the working position so as to form lateral gaps (5) at least in the distal area, and these lateral gaps (5) between the spatula parts (3a, 3b) in the working position of the spatula parts (3a, 3b) being at least substantially covered by pivotable flap elements (6), **characterized in that** the pivotable flap elements (6) on each side are each secured to at least one of the spatula parts (3a, 3b) in such a way that, in the initial position of the spatula parts (3a, 3b), they bear in a form-fitting manner on the outside of the other spatula part (3b, 3a) not provided with the respective flap element (6).

2. Spreadable medical instrument according to Claim 1, **characterized in that** the pivoting of the flap elements (6) is effected automatically via the mutually adjustable spatula parts (3a, 3b).

3. Spreadable medical instrument according to Claim 1 or 2, **characterized in that** the flap elements (6) are secured to the spatula parts in a releasable manner.

4. Spreadable medical instrument according to at least one of Claims 1 to 3, with two mutually adjustable spatula parts (3a, 3b), **characterized in that** one spatula part (3a) is connected rigidly to the grip part (2), and the other spatula part (3b) is connected to the grip part (2) so as to be adjustable in parallel and at an angle with respect to the rigid spatula part (3a), and **in that** a pivotable flap element (6) is arranged on a long side of the rigid spatula part (3a).

5. Spreadable medical instrument according to at least one of Claims 1 to 4, **characterized in that** the pivotable flap elements (6) are spring-loaded into the initial position bearing on the spatula parts (3a, 3b).

6. Spreadable medical instrument according to at least one of Claims 1 to 5, **characterized in that** the height of the pivotable flap elements (6) is such that at least part of each flap element (6), in the working position of the spatula parts (3a, 3b), bears on the outer surface of the spatula part (3b) not provided with the respective flap element (6).

7. Spreadable medical instrument according to at least one of Claims 1 to 6, **characterized in that** the outer surfaces of the spatula parts (3a, 3b) and/or of the flap elements (6) are smooth.

8. Spreadable medical instrument accoding to at least one of Claims 1 to 7, **characterized in that** the instrument is a laryngoscope (1).

## Revendications

1. Instrument médical écartable, comportant une partie de poignée (2) et au moins deux parties de spatule (3a, 3b) reliées à la partie de poignée (2) et déplaçables parallèlement et/ou angulairement l'une par rapport à l'autre via au moins un mécanisme de réglage (4) entre une position initiale dans laquelle les parties de spatule (3a, 3b) s'appuient l'une contre l'autre et au moins une position de travail, les parties de spatule individuelles (3a, 3b) étant écartées l'une de l'autre dans la position de travail en formant des intervalles latéraux (5) au moins dans la zone distale, et ces intervalles latéraux (5) entre les parties de spatule (3a, 3b) dans la position de travail des parties de spatule (3a, 3b) pouvant être recouverts au moins très largement par des éléments de volet basculants (6),
**caractérisé en ce que**
les éléments de volet basculants (6) de chaque côté sont immobilisés chacun sur l'une au moins des parties de spatule (3a, 3b) de manière à s'appuyer, dans la position initiale des parties de spatule (3a, 3b), par coopération de formes sur la face extérieure de l'autre partie de spatule respective (3b, 3a) qui n'est pas pourvue de l'élément de volet respectif (6).

2. Instrument médial écartable selon la revendication 1, **caractérisé en ce que** le basculement des éléments de volet (6) s'effectue automatiquement via les parties de spatule (3a, 3b) déplaçables l'une par rapport à l'autre.

3. Instrument médial écartable selon l'une ou l'autre des revendications 1 et 2, **caractérisé en ce que** les éléments de volet (6) sont immobilisés de façon détachable sur les parties de spatule.

4. Instrument médial écartable selon l'une au moins dès revendications 1 à 3, comportant deux parties de spatule (3a, 3b) déplaçables l'une par rapport à l'autre, **caractérisé en ce qu'**une partie de spatule (3a) est reliée rigidement à la partie de poignée (2) et l'autre partie de spatule (3b) est reliée à la partie de poignée (2) de façon déplaçable parallèlement et angulairement par rapport à la partie de spatule rigide (3a), et **en ce qu'**un élément de volet basculant respectif (6) est agencé sur un côté longitudinal de la partie de spatule rigide (3a).

5. Instrument médial écartable selon l'une au moins des revendications 1 à 4, **caractérisé en ce que** les éléments de volet basculants (6) sont sollicités par un ressort vers la position initiale en appui contre les parties de spatule (3a, 3b).

6. Instrument médial écartable selon l'une au moins des revendications 1 à 5, **caractérisé en ce que** la hauteur des éléments de volets basculants (6) est dimensionnée de telle sorte qu'une partie au moins de chaque élément de volet (6) s'appuie, dans la position de travail des parties de spatule (3a, 3b), contre la surface extérieure de la partie de spatule (3b) qui n'est pas pourvue de l'élément de volet respectif (6).

7. Instrument médial écartable selon l'une au moins des revendications 1 à 6, **caractérisé en ce que** les surfaces extérieures des parties de spatule (3a, 3b) et/ou des éléments de volet (6) sont réalisées lisses.

8. Instrument médial écartable selon l'une au moins des revendications 1 à 7, **caractérisé en ce que** l'instrument est un laryngoscope (1).
